(19) Europäisches Patentamt
European Patent Office
Office européen des brevets

(11) Veröffentlichungsnummer: **0 148 431**
**A1**

(12) # EUROPÄISCHE PATENTANMELDUNG

(21) Anmeldenummer: **84114999.0**

(22) Anmeldetag: **08.12.84**

(51) Int. Cl.⁴: **C 07 D 235/18**, A 61 K 31/415

(30) Priorität: **23.12.83 DE 3346575**

(43) Veröffentlichungstag der Anmeldung: **17.07.85**
**Patentblatt 85/29**

(84) Benannte Vertragsstaaten: **AT BE CH DE FR GB IT LI LU NL SE**

(71) Anmelder: **Dr. Karl Thomae GmbH, Postfach 1755, D-7950 Biberach (Riss) (DE)**

(72) Erfinder: **Hauel, Norbert, Dr. Dipl.-Chem., Händelstrasse 12, D-7950 Biberach 1 (DE)**
Erfinder: **Müller, Erich, Dr. Dipl.-Chem., Talfeldstrasse 34, D-7950 Biberach 1 (DE)**
Erfinder: **Austel, Volkhard, Dr. Dipl.-Chem., Kapellenweg 7, D-7950 Biberach 1 (DE)**
Erfinder: **Heider, Joachim, Dr. Dipl.-Chem., Am Hang 3, D-7951 Warthausen 1 (DE)**
Erfinder: **Reiffen, Manfred, Dr. Dipl.-Chem., Matthias-Erzberger-Strasse 40, D-7950 Biberach 1 (DE)**
Erfinder: **Diederen, Willi, Dr. Pharm., Haldenstrasse 1a, D-7950 Biberach 1 (DE)**
Erfinder: **van Meel, Jacobus Constantinus A., Dr. Pharm., Amriswilstrasse 7, D-7950 Biberach 1 (DE)**

(54) **Neue Benzimidazole, ihre Herstellung und diese Verbindungen enthaltende Arzneimittel.**

(57) Die Erfindung betrifft neue Benzimidazole der allgemeinen Formel

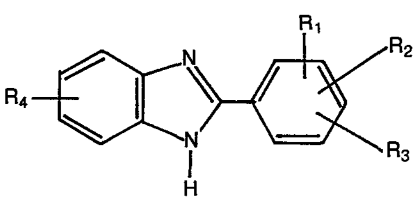

(I)

in der
$R_1$ eine Hydroxy-, Alkoxy-, Phenylalkoxy-, Cyanalkoxy-, Alkylsulfenyl-, Alkylsulfinyl-, Alkylsulfonyl-, Aminosulfonyl-, Alkylaminosulfonyl-, Dialkylaminosulfonyl-, Alkylsulfoximino-, Alkenyloxy- oder Alkinyloxygruppe,
$R_2$ ein Wasserstoffatom, eine Hydroxy-, Alkoxy-, Phenylalkoxy-, Amino-, Alkylamino- oder Dialkylaminogruppe,
$R_3$ ein Wasserstoffatom oder eine Alkoxygruppe und
$R_4$ ein Wasserstoff- oder Halogenatom, eine Cyan-, Nitro-, Amino-, Carboxy-, Alkoxycarbonyl-, Aminocarbonyl-, Alkylaminocarbonyl-, Dialkylaminocarbonyl-, Aminocarbonylamino-, Alkylaminocarbonylamino- oder Dialkylaminocarbonylaminogruppe bedeuten, deren Tautomere und deren Säureadditionssalze, welche wertvolle pharmakologische Eigenschaften aufweisen, insbesondere eine Wirkung auf den Blutdruck und auf die Kontraktilität des Herzmuskels.

Die neuen Verbindungen lassen sich nach für analoge Verbindungen bekannten Verfahren herstellen.

EP 0 148 431 A1

DR. KARL THOMAE GMBH
D-7950 Biberach 1

Case 5/896
Dr.Fl./Kp.

# Neue Benzimidazole, ihre Herstellung und diese Verbindungen enthaltende Arzneimittel

Gegenstand der vorliegenden Erfindung sind neue Benzimidazole der allgemeinen Formel

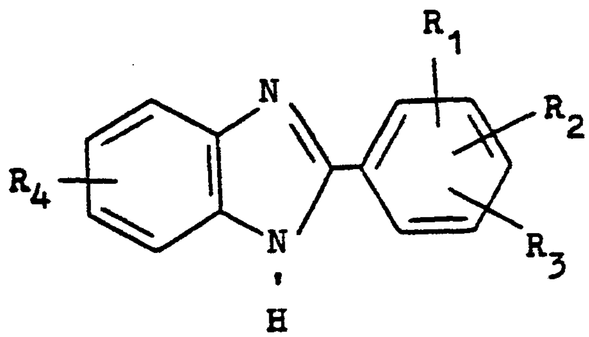

,(I)

deren Tautomere und deren Säureadditionssalze, insbesondere deren physiologisch verträgliche Säureadditionssalze mit anorganischen oder organischen Säuren, welche wertvolle pharmakologische Eigenschaften aufweisen, insbesondere eine Wirkung auf den Blutdruck und auf die Kontraktilität des Herzmuskels, diese Verbindungen enthaltende Arzneimittel und Verfahren zu ihrer Herstellung.

In der obigen allgemeinen Formel I bedeutet

$R_1$ eine Hydroxy-, Alkoxy-, Phenylalkoxy-, Cyanalkoxy-, Alkylsulfenyl-, Alkylsulfinyl-, Alkylsulfonyl-, Aminosulfonyl-, Alkylaminosulfonyl-, Dialkylaminosulfonyl-, Alkylsulfoximino-, Alkenyloxy- oder Alkinyloxygruppe,

$R_2$ ein Wasserstoffatom, eine Hydroxy-, Alkoxy-, Phenylalkoxy-, Amino-, Alkylamino- oder Dialkylaminogruppe,

$R_3$ ein Wasserstoffatom oder eine Alkoxygruppe und

$R_4$ ein Wasserstoff- oder Halogenatom, eine Cyan-, Nitro-, Amino-, Carboxy-, Alkoxycarbonyl-, Aminocarbonyl-, Alkylaminocarbonyl-, Dialkylaminocarbonyl-, Aminocarbonylamino-, Alkylaminocarbonylamino- oder Dialkylaminocarbonylaminogruppe, wobei der Alkylteil jeweils 1 bis 3 Kohlenstoffatome und der Alkenyl- oder Alkinylteil jeweils 3 bis 5 Kohlenstoffatome enthalten kann.

Für die bei der Definition der Reste $R_1$ bis $R_4$ eingangs erwähnten Bedeutungen kommt beispielsweise

für $R_1$ die Bedeutung der Hydroxy-, Methoxy-, Ethoxy-, n-Propoxy-, Benzyloxy-, 1-Phenylethoxy-, 2-Phenylethoxy-, 3-Phenylpropoxy-, Methylsulfenyl-, Ethylsulfenyl-, Isopropylsulfenyl-, Methylsulfinyl-, Ethylsulfinyl-, n-Propylsulfinyl-, Methylsulfonyl-, Ethylsulfonyl-, n-Propylsulfonyl-, Isopropylsulfonyl-, Cyanmethoxy-, 2-Cyanethoxy-, 3-Cyanpropoxy-, Aminosulfonyl-, Methylaminosulfonyl-, Ethylaminosulfonyl-, n-Propylaminosulfonyl-, Dimethylaminosulfonyl-, Diethylaminosulfonyl-, Diisopropylaminosulfonyl-, N-Methylethylaminosulfonyl-, Methylsulfoximino-, Ethylsulfoximino-, n-Propylsulfoximino-, Allyloxy-, But-2-enyloxy-, Pent-2-enyloxy-, Propargyloxy-, But-2-inyloxy- oder Pent-2-inyloxygruppe,

für $R_2$ die des Wasserstoffatoms, der Methoxy-, Ethoxy-, n-Propoxy-, Isopropoxy-, Benzyloxy-, 1-Phenylethoxy-, 2-Phenylethoxy-, 3-Phenylpropoxy-, Amino-, Methylamino-, Ethylamino-, Isopropylamino-, Dimethylamino-, Diethylamino-, Di-n-propylamino-, Diisopropylamino-, N-Methyl-ethylamino-, N-Methyl-isopropylamino- oder N-Ethyl-n-propylaminogruppe,

für $R_3$ die des Wasserstoffatoms, der Methoxy-, Ethoxy-, n-Propoxy- oder Isopropoxygruppe und

für $R_4$ die des Wasserstoff-, Fluor-, Chlor- oder Bromatoms, der Cyan-, Nitro-, Amino-, Carboxy-, Methoxycarbonyl-, Ethoxycarbonyl-, n-Propoxycarbonyl-, Aminocarbonyl-, Methylaminocarbonyl-, Ethylaminocarbonyl-, n-Propylaminocarbonyl-, Dimethylaminocarbonyl-, Diethylaminocarbonyl-, Diisopropylaminocarbonyl-, Aminocarbonylamino-, Methylaminocarbonylamino-, Ethylaminocarbonylamino-, n-Propylaminocarbonylamino-, Dimethylaminocarbonylamino-, Diethylaminocarbonylamino-, Di-n-propylaminocarbonylamino- oder N-Methylethylaminocarbonylaminogruppe in Betracht.

Bevorzugte Verbindungen der obigen allgemeinen Formel I sind diejenigen, in denen
$R_1$ und $R_4$ wie eingangs definiert sind,
$R_2$ mit Ausnahme des Wasserstoffatoms die für $R_2$ eingangs erwähnten Bedeutungen besitzt und
$R_3$ ein Wasserstoffatom darstellt,

insbesondere diejenigen Verbindungen, in denen
$R_1$ in 4-Stellung und $R_2$ in 2-Stellung steht
und
$R_4$ mit Ausnahme des Wasserstoffatoms die für $R_4$ eingangs erwähnten Bedeutungen besitzt.

Besonders bevorzugte Verbindungen sind die Verbindungen der allgemeinen Formel

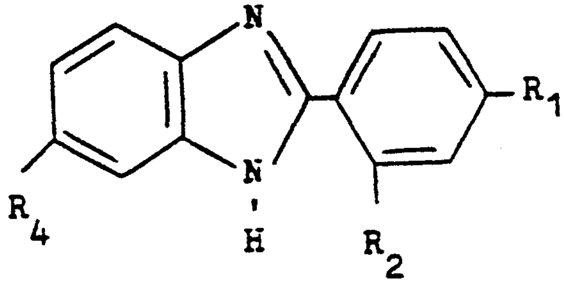

,(Ia)

in der
$R_1$ eine Hydroxy-, Benzyloxy-, Allyloxy-, Propargyloxy-, Alkylsulfenyl-, Alkylsulfinyl-, Alkylsulfonyl-, Alkylsulfoximino- oder Aminosulfonylgruppe,

$R_2$ eine Alkoxygruppe und

$R_4$ ein Fluor-, Chlor- oder Bromatom, eine Cyan-, Carboxy-, Aminocarbonyl-, Alkoxycarbonyl-, Nitro- oder Aminogruppe bedeuten, wobei der Alkylteil jeweils 1 oder 2 Kohlenstoffatome enthalten kann, deren Tautomere und deren Säureadditionssalze, insbesondere deren physiologisch verträgliche Säureadditionssalze mit anorganischen oder organischen Säuren.

Erfindungsgemäß erhält man die neuen Verbindungen nach folgenden Verfahren:

a) Cyclisierung einer gegebenenfalls im Reaktionsgemisch hergestellten Verbindung der allgemeinen Formel

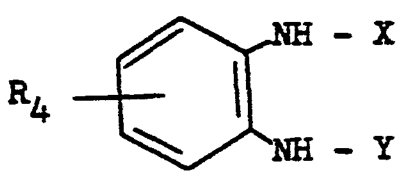
,(II)

in der
$R_4$ wie eingangs definiert ist,
einer der Reste X oder Y ein Wasserstoffatom und der andere der beiden Reste X und Y oder beide Reste X und Y eine Gruppe der Formel

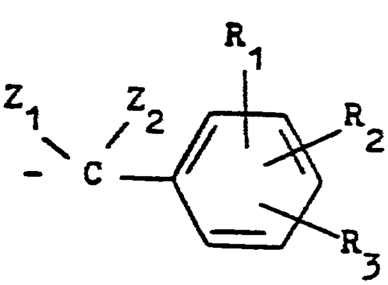
darstellen, in der

$R_1$ bis $R_3$ wie eingangs definiert sind, $Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder

$Z_1$ und $Z_2$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten.

Die Cyclisierung wird zweckmäßigerweise in einem Lösungsmittel oder Lösungsmittelgemisch wie Ethanol, Isopropanol, Eisessig, Benzol, Chlorbenzol, Toluol, Xylol, Glycol, Glycolmonomethylether, Diethylenglycoldimethylether, Sulfolan, Dimethylformamid, Tetralin oder in einem Überschuß des zur Herstellung der Verbindung der allgemeinen Formel II verwendeten Acylierungsmittel, z.B. in dem entsprechenden Nitril, Anhydrid, Säurehalogenid, Ester, Amid oder Methojodid, beispielsweise bei Temperaturen zwischen 0 und 250°C, vorzugsweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Salzsäure, Phosphorsäure, Polyphosphorsäure, Essigsäureanhydrid oder gegebenenfalls auch in Gegenwart einer Base wie Kaliumäthylat oder Kalium-tert.butylat durchgeführt. Die Cyclisierung kann jedoch auch ohne Lösungsmittel und/oder Kondensationsmittel durchgeführt werden.

b) Zur Herstellung von Verbindungen der allgemeinen Formel I, in der $R_1$ eine Alkylsulfinyl- oder Alkylsulfonylgruppe darstellt:

Oxidation einer Verbindung der allgemeinen Formel

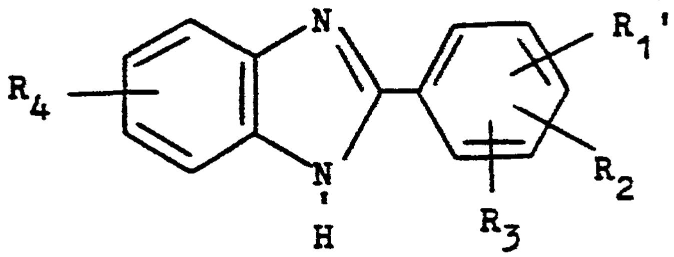

,(III)

in der
$R_2$ bis $R_4$ wie eingangs definiert sind und
$R_1$' eine Alkylsulfenyl- oder Alkylsulfinylgruppe mit jeweils 1 bis 3 Kohlenstoffatomen im Alkylteil darstellt.

Die Oxidation wird vorzugsweise in einem Lösungsmittel oder Lösungsmittelgemisch, z.B. in Wasser, Wasser/Pyridin, Aceton, Eisessig, verdünnter Schwefelsäure oder Trifluoressigsäure, je nach dem verwendeten Oxidationsmittel zweckmäßigerweise bei Temperaturen zwischen -80 und 100°C durchgeführt.

Zur Herstellung einer Alkylsulfinylverbindung der allgemeinen Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Methylenchlorid oder Chloroform bei -20 bis 60°C, mit Natriummetaperjodat in wässrigem Methanol oder Äthanol bei -15 bis 25°C, mit Brom in Eisessig oder wässriger Essigsäure, mit N-Brom-succinimid in Äthanol, mit tert.Butyl-hyprochlorit in Methanol bei -80 bis -30°C, mit Jodbenzodichlorid in wässrigem Pyridin bei 0 bis 50°C,

mit Salpetersäure in Eisessig bei 0 bis 20°C, mit Chromsäure in Eisessig oder in Aceton bei 0 bis 20°C und mit Sulfurylchlorid in Methylenchlorid bei -70°C, der hierbei erhaltene Thioäther-Chlor-Komplex wird zweckmäßigerweise mit wäßrigem Äthanol hydrolysiert.

Zur Herstellung einer Alkylsulfonylverbindung der allgemeinen Formel I wird die Oxidation zweckmäßigerweise mit einem bzw. mit zwei oder mehr Äquivalenten des verwendeten Oxidationsmittels durchgeführt, z.B. mit Wasserstoffperoxid in Eisessig, Trifluoressigsäure oder in Ameisensäure bei 20 bis 100°C oder in Aceton bei 0 bis 60°C, mit einer Persäure wie Perameisensäure oder m-Chlorperbenzoesäure in Eisessig, Trifluoressigsäure, Methylenchlorid oder Chloroform bei Temperaturen zwischen 0 und 60°C, mit Salpetersäure in Eisessig bei 0 bis 20°C, mit Chromsäure oder Kaliumpermanganat in Eisessig, Wasser/Schwefelsäure oder in Aceton bei 0 bis 20°C.

c) Zur Herstellung von Verbindungen der allgemeinen Formel I, in der $R_1$ eine Alkoxy-, Phenylalkoxy-, Cyanalkoxy-, Alkylsulfenyl-, Alkenyloxy- oder Alkinyloxygruppe darstellt:

Umsetzung einer Verbindung der allgemeinen Formel

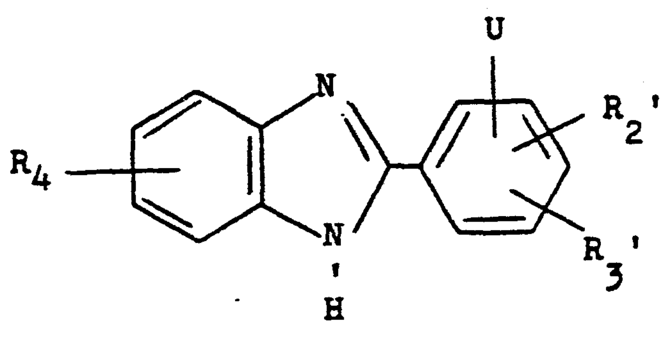

,(IV)

in der

$R_4$ wie eingangs definiert ist,

U eine Hydroxy- oder Mercaptogruppe darstellt,

$R_2$' eine Hydroxygruppe oder die für $R_2$ eingangs erwähnten Bedeutungen besitzt und

$R_3$' eine Hydroxygruppe oder die für $R_3$ eingangs erwähnten Bedeutungen besitzt, mit einem Halogenid der allgemeinen Formel

$$W - R_5 \qquad ,(V)$$

in der
$R_5$ eine Alkyl-, Phenylalkyl-, Cyanalkyl-, Alkenyl- oder Alkinylgruppe, wobei der Alkylteil jeweils 1 bis 3 Kohlenstoffatomen und der Alkenyl- bzw. Alkinylteil jeweils 3 bis 5 Kohlenstoffatomen enthalten kann, und
W eine nukleophile Austrittsgruppe wie ein Chlor-, Brom- oder Jodatom darstellen.

Die Umsetzung wird zweckmäßigerweise in einem Lösungsmittel wie Tetrahydrofuran, Dioxan, Dimethylformamid, Sulfolan, Dimethylsulfoxid oder Ethylenglycoldimethylether vorzugsweise in Gegenwart eines säurebindenden Mittels wie Kaliumkarbonat, Kalium-tert.butylat oder Natriumhydrid bei Temperaturen zwischen 0 und 100°C, vorzugsweise jedoch bei Temperaturen zwischen 20 und 50°C, durchgeführt.

d) Zur Herstellung von Verbindungen der allgemeinen Formel I, in der $R_4$ eine Alkoxcarbonyl-, Aminocarbonyl-, Alkylaminocarbonyl- oder Dialkylaminocarbonylgruppe darstellt:

Umsetzung eines Benzimidazols der allgemeinen Formel

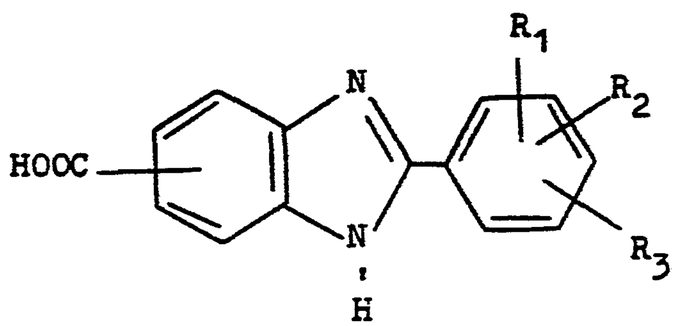

,(VI)

in der
$R_1$ bis $R_3$ wie eingangs definiert sind, oder deren gegebenenfalls im Reaktionsgemisch hergestelltes reaktionsfähiges Derivat mit einer Verbindung der allgemeinen Formel

$$H - R_6 \quad ,(VII)$$

in der
$R_6$ eine Alkoxy-, Amino-, Alkylamino- oder Dialkylaminogruppe darstellt, wobei der Alkylteil jeweils 1 bis 3 Kohlenstoffatomen enthalten kann, oder mit einem gegebenenfalls im Reaktionsgemisch gebildeten N-aktivierten Amin der allgemeinen Formel VII, wenn eine Carbonsäure der allgemeinen Formel VI eingesetzt wird.

Das Verfahren betrifft somit die Acylierung einer Verbindung der allgemeinen Formel VII mit einer Carbonsäure der allgemeinen Formel VI in Gegenwart eines die Säure aktivierenden oder eines wasserentziehenden Mittels oder mit deren funktionellen Derivaten oder
die Umsetzung einer Carbonsäure der allgemeinen Formel VI mit einem Amin der allgemeinen Formel VII in Gegenwart eines die Aminogruppe aktivierenden Mittels oder mit dessen reaktionsfähigen Derivaten.

Als gegebenenfalls im Reaktionsgemisch hergestellte funktionelle Derivate einer Carbonsäure der allgemeinen Formel VI kommen beispielsweise deren Alkyl-, Aryl- oder Aralkylester oder -thioester wie der Methyl-, Äthyl-, Phenyl- oder Benzylester, deren Imidazolide, deren Säurehalogenide wie das Säurechlorid oder -bromid, deren Anhydride, deren gemischte Anhydride mit aliphatischen oder aromatischen Carbon-, Sulfen-, Sulfin- oder Sulfonsäuren oder Kohlensäureestern, z.B. der Essigsäure, Propionsäure, p-Toluolsulfonsäure oder der O-Äthyl-kohlensäure, deren O-Triphenylphosphonium-Komplexe, deren N-Acyl-oxyimide, deren Azide oder Nitrile oder die entsprechenden Amino-thiocarbonsäure-Derivate, und als gegebenenfalls im Reaktionsgemisch hergestellte reaktionsfähige Derivate eines Amins der allgemeinen Formel VI deren Phosphazoderivate in Betracht.

Als säureaktivierende und/oder wasserentziehende Mittel kommen beispielsweise ein Chlorameisensäureester wie Chlorameisensäureäthylester, Thionylchlorid, Phosphortrichlorid, Phosphorpentoxid, N,N'-Dicyclohexylcarbodiimid, N,N'-Carbonyldiimidazol, N,N'-Thionyldiimidazol, Bortrifluoridätherat oder Triphenylphosphin/Tetrachlorkohlenstoff in Betracht.

Die Umsetzung wird zweckmäßigerweise in einem Lösungmittel wie Methylenchlorid, Chloroform, Tetrachlorkohlenstoff, Äther, Tetrahydrofuran, Dioxan, Benzol, Toluol, Acetonitril oder Dimethylformamid, gegebenenfalls in Gegenwart einer anorganischen Base wie Natriumkarbonat oder einer tertiären organischen Base wie Triäthylamin oder Pyridin, welche gleichzeitig als Lösungsmittel dienen kann, und gegebenenfalls in Gegenwart eines säureaktivierenden Mittels bei Temperaturen zwischen -25 und 250°C, vorzugsweise jedoch bei Temperaturen zwischen -10°C und der Siedetemperatur des verwendeten Lösungsmittels, durchgeführt. Hierbei braucht ein gegebenenfalls im Reaktionsgemisch entstandenes funktionelles Derivat einer Verbindung der allgemeinen Formel VI oder VII nicht isoliert zu werden, ferner kann die Umsetzung auch ohne Lösungsmittel durchgeführt werden. Desweiteren kann während der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

e) Zur Herstellung von Verbindungen der allgemeinen Formel I, in der $R_1$ eine Alkylsulfoximinogruppe darstellt:

Umsetzung eines Sulfoxids der allgemeinen Formel

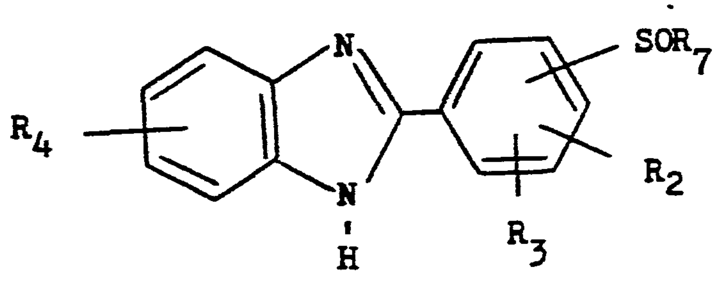

,(VIII)

in der
$R_2$ bis $R_4$ wie eingangs definiert sind und
$R_7$ eine Alkylgruppe mit 1 bis 3 Kohlenstoffatomen darstellt, mit gegebenenfalls im Reaktionsgemisch gebildeter Stickstoffwasserstoffsäure.

Die Umsetzung wird zweckmäßigerweise in einem Lösungsmittel oder Lösungsmittelgemisch wie Methylenchlorid, Dimethylformamid oder Tetrahydrofuran bei Temperaturen zwischen 0 und 40°C, vorzugsweise bei Temperaturen zwischen 10 und 35°C, durchgeführt. Besonders vorteilhaft wird die Umsetzung mit einem Alkaliazid, z.B. Natriumazid, und Polyphosphorsäure als Lösungsmittel durchgeführt.

f) Zur Herstellung von Verbindungen der allgemeinen Formel I, in der $R_1$ eine Alkylsulfoximinogruppe darstellt:

Umsetzung eines Sulfoxids der allgemeinen Formel

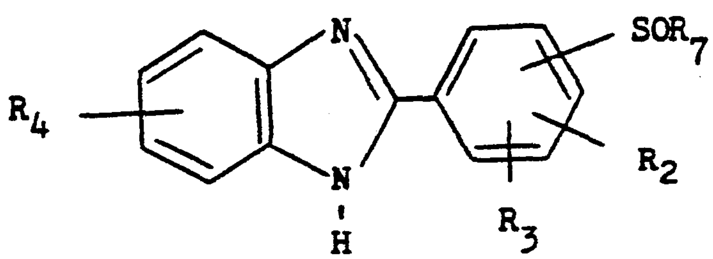

,(VIII)

in der
$R_2$ bis $R_4$ wie eingangs definiert sind und
$R_7$ eine Alkylgruppe mit 1 bis 3 Kohlenstoffatomen darstellt, mit einer Verbindung der allgemeinen Formel

$$H_2N - O - W - R_8 \quad ,(IX)$$

in der
W eine Carbonyl- oder Sulfonylgruppe und
$R_8$ eine in o-Stellung disubstituierte Arylgruppe wie eine 2,4,6-Trimethylphenyl- oder 2,4,6-Triisopropylphenylgruppe darstellen.

Die Umsetzung wird zweckmäßigerweise in einem Lösungsmittel oder Lösungsmittelgemisch wie Methylenchlorid, Chloroform, Dimethylformamid, Tetrahydrofuran oder Dioxan bei Temperaturen zwischen 0 und 50°C, vorzugsweise jedoch bei Temperaturen zwischen 5 und 40°C, und gegebenenfalls in Gegenwart einer katalytischen Menge einer Säure wie p-Toluolsulfonsäure durchgeführt. Besonders vorteilhaft wird die Umsetzung jedoch in der Weise durchgeführt, daß eine Verbindung der allgemeinen Formel IX ohne ihre vorherige Isolierung eingesetzt bzw. im Reaktionsgemisch hergestellt wird.

Erhält man erfindungsgemäß eine Verbindung der allgemeinen Formel I, in der $R_4$ eine Alkoxycarbonyl-, Cyan-, Aminocarbonyl-, Alkylaminocarbonyl- oder Dialkylaminocarbonylgruppe darstellt, so kann diese mittels Hydroylse in eine entsprechende Verbindung der allgemeinen Formel I, in der $R_4$ die Carboxylgruppe darstellt, übergeführt werden und/oder

eine Verbindung der allgemeinen Formel I, in der $R_4$ eine Aminocarbonylgruppe darstellt, so kann diese mittels Dehydratisierung in eine entsprechende Verbindung der allgemeinen Formel I, in der $R_4$ die Cyangruppe darstellt, übergeführt werden und/oder

eine Verbindung der allgemeinen Formel I, in der $R_4$ eine Nitrogruppe darstellt, so kann diese mittels Reduktion in eine entsprechende Verbindung der allgemeinen Formel I, in der $R_4$ eine Aminogruppe darstellt, übergeführt werden und/oder

eine Verbindung der allgemeinen Formel I, in der $R_4$ eine Aminogruppe darstellt, so kann diese mittels Carbamoylierung in eine entsprechende Verbindung der allgemeinen Formel I, in der $R_4$ eine Aminocarbonylamino-, Alkylaminocarbonylamino- oder Dialkylaminocarbonylaminogruppe darstellt, übergeführt werden und/oder

eine Verbindung der allgemeinen Formel I, in der $R_1$ und/oder $R_2$ eine Benzyloxygruppe darstellen, so kann diese mittels Entbenzylierung in eine entsprechende Verbindung der allgemeinen Formel I, in der $R_1$ und/oder $R_2$ eine Hydroxygruppe darstellen, übergeführt werden.

Die nachträgliche Hydrolyse wird zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Wasser/Methanol, Ethanol, Wasser/Ethanol, Wasser/Isopropanol oder Wasser/Dioxan bei Temperaturen zwischen -10 und 120°C, z.B. bei Temperaturen zwischen Raumtemperaur und der Siedetemperatur des Reaktionsgemisches, durchgeführt, die partielle Hydrolyse wird jedoch vorzugsweise mit konzentrierter Schwefelsäure durchgeführt.

Die nachträgliche Dehydratisierung wird mit einem wasserentziehenden Mittel wie Phosphorpentoxid, Phosphoroxichlorid, Schwefelsäure oder p-Toluolsulfonsäurechlorid gegebenenfalls in einem Lösungsmittel wie Methylenchlorid oder Pyridin bei Temperaturen zwischen 0 und 100°C, vorzugsweise bei Temperaturen zwischen 20 und 80°C, durchgeführt.

Die nachträgliche Reduktion der Nitrogruppe wird vorzugsweise in einem Lösungsmittel wie Wasser, Wasser/Ethanol, Methanol, Eisessig, Essigsäureethylester oder Dimethylformamid zweckmäßigerweise mit Wasserstoff in Gegenwart eines Hydrierungskatalysators wie Raney-Nickel, Platin oder Palladium/Kohle, mit Metallen wie Eisen, Zinn oder Zink in Gegenwart einer Säure, mit Salzen wie Eisen(II)sulfat, Zinn(II)chlorid, Natriumsulfid, Natriumhydrogensulfid oder Natriumdithionit, oder mit Hydrazin in Gegenwart von Raney-Nickel bei Temperaturen zwischen 0 und 50°C, vorzugsweise jedoch bei Raumtemperatur, durchgeführt.

Die nachträgliche Carbamoylierung wird in einem inerten Lösungsmittel wie Wasser, Methylenchlorid, Tetrahydrofuran oder Dioxan mit einem entsprechenden Isocyanat wie Methylisocyanat oder Kaliumisocyanat in Gegenwart einer Säure wie Essigsäure oder mit einem entsprechenden Carbamoylchlorid wie Dimethylaminocarbonylchlorid bei Temperaturen zwischen 0 und 50°C durchgeführt.

Die nachträgliche Entbenzylierung wird zweckmäßigerweise in einem Lösungmittel wie Methanol, Äthanol, Essigester oder Eisessig mittels katalytisch angeregtem Wasserstoff, z.B. mit Wasserstoff in Gegenwart von Platin oder Palladium/Kohle, bei Temperaturen zwischen 0 und 75°C, vorzugsweise jedoch bei Raumtemperatur, und bei einem Wasserstoffdruck von 1-5 bar durchgeführt.

Ferner können die erhaltenen Verbindungen der allgemeinen Formel I anschließend gewünschtenfalls in ihre physiologisch verträglichen Säureadditionssalze mit anorganischen oder organischen Säuren übergeführt werden. Als Säuren kommen hierfür beispielsweise Salzsäure, Bromwasserstoffsäure, Schwefelsäure, Phosphorsäure, Furmarsäure, Bernsteinsäure, Weinsäure, Zitronensäure, Milchsäure, Maleinsäure oder Methansulfonsäure in Betracht.

Die als Ausgangsstoffe verwendeten Verbindungen der allgemeinen Formeln II bis IX sind teilweise literaturbekannt bzw. erhält man nach literaturbekannten Verfahren.

So erhält man beispielsweise die als Ausgangsstoffe verwendeten Verbindungen der allgemeinen Formel II durch Acylierung einer entsprechenden o-Diaminoverbindung und die Verbindungen der allgemeinen Formeln III, IV, VI und VIII durch anschließende Kondensation mit einem entsprechenden Benzoesäurederivat und gegebenenfalls anschließende Oxidation (siehe EP-A-0.022.495).

Wie bereits eingangs erwähnt weisen die neuen Verbindungen der allgemeinen Formel I, deren 1H Tautomere und deren physiologisch verträgliche Säureadditionssalze bei einer langen Wirkungsdauer überlegene pharmakologische Eigenschaften auf, insbesondere eine blutdrucksenkende, positiv-inotrope und/oder antithrombotische Wirkung.

Beispielsweise wurden die Verbindungen

A = 2-(2-Methoxy-4-methylsulfonyl-phenyl)-5-cyano-benzimidazol und

B = 2-(2-Methoxy-4-propargyloxy-phenyl)-5-cyano-benzimidazol

auf ihre biologischen Eigenschaften wie folgt untersucht:

Bestimmung der Blutdruckwirkung und der positiv inotropen Wirkung an der narkotisierten Katze

Die Untersuchungen wurden an Katzen durchgeführt, die mit Pentobarbital-Natrium (40 mg/kg i.p.) narkotisiert waren. Die Tiere atmeten spontan. Der arterielle Blutdruck wurde in der Aorta abdominalis mit einem Statham-Druckwandler (P 23 Dc) gemessen. Für die Erfassung der positiv inotropen Wirkung wurde mit einem Kathetertipmanometer (Millar PC-350 A) der Druck in der linken Herzkammer gemessen. Daraus wurde der Kontraktilitätsparameter $dp/dt_{max}$ mittels eines Analogdifferenzierers gewonnen. Die zu untersuchenden Substanzen wurden in eine Vena femoralis injiziert. Als Lösungsmittel diente Polydiol 200. Jede Substanz wurde an mindestens 3 Katzen geprüft.

Die nachfolgende Tabelle enthält die gefundenen Mittelwerte:

| Substanz | Dosis mg/kg i.v. | Zunahme von dp/$dt_{max}$ in % | Blutdrucksenkung in mm Hg |
|---|---|---|---|
| A | 0,2 | 51 | - 29/-23 |
| A | 0,6 | 96 | - 36/-37 |
| A | 2,0 | 79 | - 49/-48 |
| B | 0,2 | 33 | - 40/-27 |
| B | 0,6 | 48 | - 48/-37 |
| B | 2,0 | 85 | - 57/-33 |

Die neuen Verbindungen sind gut verträglich, so konnte bei der Untersuchung der Substanzen A und B keinerlei herztoxische Wirkungen bzw. Kreislaufschäden beobachtet werden.

Aufgrund ihrer pharmakologischen Eigenschaften eignen sich die erfindungsgemäß hergestellten Verbindungen der allgemeinen Formel I sowie deren physiologisch verträgliche Säureadditionssalze zur Behandlung von Herzinsuffizienzen unterschiedlicher Genese, da sie die Kontraktionskraft des Herzens steigern und durch die Blutdrucksenkung die Entleerung des Herzens erleichtern.

Hierzu lassen sich die neuen Verbindungen sowie deren physiologisch verträgliche Säureadditionssalze, gegebenenfalls in Kombination mit anderen Wirksubstanzen, in die üblichen pharmazeutischen Anwendungsformen wie Tabletten, Dragées, Pulver, Suppositorien, Suspensionen, Ampullen oder Tropfen einarbeiten. Die Einzeldosis beträgt hierbei 1-4 x täglich 0,3 - 2,2 mg/kg Körpergewicht, vorzugsweise jedoch 0,7 - 1,5 mg/kg Körpergewicht.

Die nachfolgenden Beispiele sollen die Erfindung näher erläutern:

Beispiel 1

2-(2-Methoxy-4-methylmercapto-phenyl)-5-methoxycarbonyl-benzimidazol

23,9 g 4-Methoxycarbonyl-1,2-phenylendiamin-dihydrochlorid und 19,8 g 2-Methoxy-4-methylmercapto-benzoesäure werden mit 700 ml Phosphoroxychlorid 2,5 Stunden am Rückfluß erhitzt. Anschließend wird das überschüssige Phosphoroxychlorid im Vakuum abdestilliert, der kristalline Rückstand mit Eiswasser verrührt, mit konzentriertem Ammoniak bis zur alkalischen Reaktion versetzt und die Kristalle abgesaugt. Man kristallisiert aus 70%igem Äthylalkohol und trocknet bei 70°C.
Schmelzpunkt: 206-208°C,
Ausbeute: 23,0 g (70 % der Theorie).

Analog werden folgende Verbindungen erhalten:

2-(2-Methoxy-4-ethylmercapto-phenyl)-5-methoxycarbonyl-benzimidazol

2-(2-Ethoxy-4-n-propylmercapto-phenyl)-5-methoxycarbonyl-benzimidazol

2-(2-Methoxy-4-methylmercapto-phenyl)-5-propoxycarbonyl-benzimidazol

Beispiel 2

2-(2-Methoxy-4-methylmercapto-phenyl)-5-carboxy-benzimidazol

10,2 g 2-(2-Methoxy-4-methylmercapto-phenyl)-5-methoxycarbonyl-benzimidazol werden in 300 ml 2n Natronlauge 2 Stunden unter Rückfluß zum Sieden erhitzt. Anschließend wird noch

heiß über Kieselgur filtriert und mit 2n Salzsäure angesäuert. Man saugt ab, wäscht mit Wasser und trocknet bei 70°C.
Schmelzpunkt: 308-310°C (Zersetzung),
Ausbeute: 8,6 g (88 % der Theorie).

Analog werden folgende Verbindungen erhalten:

2-(2-Methoxy-4-ethylmercapto-phenyl)-5-carboxy-benzimidazol

2-(2-Ethoxy-4-n-propylmercapto-phenyl)-5-carboxy-benzimidazol

<u>Beispiel 3</u>

<u>2-(2-Methoxy-4-methylmercapto-phenyl)-5-aminocarbonyl-benzimidazol</u>

6,7 g 2-(2-Methoxy-4-methylmercapto-phenyl)-5-carboxy-benzimidazol, suspendiert in 120 ml Chloroform, werden unter Rückfluß tropfenweise mit 25,4 g Thionylchlorid unter kräftiger Gasentwicklung versetzt. Nach 2-stündigem Erhitzen kühlt man auf Raumtemperatur ab und saugt das gebildete Säurechlorid ab. Das Rohprodukt wird in 100 ml konzentriertem Ammoniak eingetragen. Man saugt das erhaltene Säureamid ab, löst in siedendheißem Ethanol und fällt mit heißem Wasser. Die Kristalle werden abgesaugt und bei 80°C getrocknet.
Schmelzpunkt: 138-142°C,
Ausbeute: 4,5 g (67,4 % der Theorie).

Analog werden folgende Verbindungen erhalten:

2-(2-Methoxy-4-methylmercapto-phenyl)-5-methylaminocarbonyl-benzimidazol

2-(2-Methoxy-4-methylmercapto-phenyl)-5-ethylaminocarbonyl-benzimidazol

2-(2-Methoxy-4-methylmercapto-phenyl)-5-dimethylaminocarbonyl-benzimidazol

2-(2-Methoxy-4-methylmercapto-phenyl)-5-di-n-propylaminocarbonyl-benzimidazol

2-(2-Methoxy-4-ethylmercapto-phenyl)-5-aminocarbonyl-benzimidazol

2-(2-Ethoxy-4-methylmercapto-phenyl)-5-methylaminocarbonyl-benzimidazol

2-(2-Ethoxy-4-ethylmercapto-phenyl)-5-diethylaminocarbonyl-benzimidazol

2-(2-Methoxy-4-n-propylmercapto-phenyl)-5-aminocarbonyl-benzimidazol

## Beispiel 4

2-(2-Methoxy-4-methylmercapto-phenyl)-5-cyano-benzimidazol

3,5 g 2-(2-Methoxy-4-methylmercapto-phenyl)-5-aminocarbonyl-benzimidazol werden mit 100 ml Phosphoroxychlorid 2,5 Stunden am Rückfluß erhitzt und anschließend das überschüssige Phosphoroxychlorid im Vakuum abdestilliert. Der Rückstand wird mit Eis/Wasser zersetzt, mit konzentriertem Ammoniak bis zur alkalischen Reaktion versetzt und abgesaugt. Die erhaltenen Kristalle werden bei 70°C getrocknet.
Schmelzpunkt: 211-215°C,
Ausbeute: 3,05 g (92,4 % der Theorie).

Analog werden folgende Verbindungen erhalten:

2-(2-Methoxy-4-ethylmercapto-phenyl)-5-cyano-benzimidazol

2-(2-Methoxy-4-n-propylmercapto-phenyl)-5-cyano-benzimidazol

2-(2-Ethoxy-4-methylmercapto-phenyl)-5-cyano-benzimidazol

Beispiel 5

2-(2-Methoxy-4-methylsulfinyl-phenyl)-5-cyano-benzimidazol

2,0 g 2-(2-Methoxy-4-methylmercapto-phenyl)-5-cyano-benzimidazol werden in 50 ml Eisessig gelöst und mit 0,61 ml Wasserstoffperoxid (399 mg/ml) versetzt. Nach 14 Stunden wird mit 50 ml Wasser und mit 3 ml einer 40%igen Natriumbisulfit-Lösung versetzt, danach zur Trockne im Vakuum eingedampft. Der Rückstand wird mit Wasser aufgenommen, abgesaugt und bei 70°C getrocknet.
Schmelzpunkt: 115-116°C,
Ausbeute: 1,79 g (84,9 % der Theorie),

Analog werden folgende Verbindungen erhalten:

2-(2-Methoxy-4-ethylsulfinyl-phenyl)-5-cyano-benzimidazol

2-(2-Methoxy-4-n-propylsulfinyl-phenyl)-5-cyano-benzimidazol

2-(2-Ethoxy-4-methylsulfinyl-phenyl)-5-cyano-benzimidazol

Beispiel 6

2-(2-Methoxy-4-methylsulfonyl-phenyl)-5-cyano-benzimidazol

0,7 g 2-(2-Methoxy-4-methylmercapto-phenyl)-5-cyano-benzimidazol werden in 10 ml Ameisensäure gelöst, mit 0,51 ml Wasserstoffperoxid (399 mg/ml) versetzt und 4 Stunden stehen gelassen. Anschließend verdünnt man mit 40 ml Wasser, saugt die abgeschiedenen Kristalle ab und trocknet bei 60°C.
Schmelzpunkt: 260-261°C,
Ausbeute: 0,71 g (91,5 % der Theorie).

Analog werden folgende Verbindungen erhalten:

2-(2-Methoxy-4-ethylsulfonyl-phenyl)-5-cyano-benzimidazol

2-(2-Methoxy-4-n-propylsulfonyl-phenyl)-5-cyano-benzimidazol

2-(2-Ethoxy-4-methylsulfonyl-phenyl)-5-cyano-benzimidazol

## Beispiel 7

2-(2-Methoxy-4-methylsulfoximino-phenyl)-5-cyano-benzimidazol

0,623 g 2-(2-Methoxy-4-methylsulfinyl-phenyl)-5-cyano-benzimidazol, gelöst in 5 ml Dimethylformamid, werden mit 1,43 g 0-Mesitylensulfonyl-acethydroxamsäureäthylester und 1,71 g p-Toluolsulfonsäure versetzt. Nach 20 Stunden versetzt man mit 10 ml Wasser und gibt konzentriertes Ammoniak bis zur alkalischen Reaktion zu. Die auskristallisierende Substanz wird aus Ethanol umkristallisiert.
Schmelzpunkt: 262-263°C (Zers.),
Ausbeute: 0,33 g (50,6 % der Theorie).

Analog wird folgende Verbindung erhalten:

2-(2-Methoxy-4-ethylsulfoximino-phenyl)-5-cyano-benzimidazol

Beispiel 8

2-(2-Methoxy-4-methylsulfinyl-phenyl)-5-carbamido-benzimidazol

Hergestellt analog Beispiel 5 aus 2-(2-Methoxy-4-methylmercapto-phenyl)-5-carbamido-benzimidazolund Wasserstoffperoxid in Eisessig.
Schmelzpunkt: 175-178°C,
Ausbeute: 53,5 % der Theorie.

Analog werden folgende Verbindungen erhalten:

2-(2-Methoxy-4-ethylsulfinyl-phenyl)-5-carbamido-benzimidazol

2-(2-Methoxy-4-n-propylsulfinyl-phenyl)-5-carbamido-benzimidazol

2-(2-Ethoxy-4-methylsulfinyl-phenyl)-5-carbamido-benzimidazol

Beispiel 9

2-(2-Methoxy-4-methylsulfonyl-phenyl)-5-carbamido-benzimidazol

Hergestellt analog Beispiel 6 aus 2-(2-Methoxy-4-methylmercapto-phenyl)-5-carbamido-benzimidazolund Wasserstoffperoxid in Ameisensäure.
Schmelzpunkt: 278-280°C,
Ausbeute: 41,6 % der Theorie.

Analog werden folgende Verbindungen erhalten:

2-(2-Methoxy-4-ethylsulfonyl-phenyl)-5-carbamido-benzimidazol

2-(2-Methoxy-4-n-propylsulfonyl-phenyl)-5-carbamido-benzimidazol

2-(2-Ethoxy-4-methylsulfonyl-phenyl)-5-carbamido-benzimidazol

## Beispiel 10

**2-(2-Methoxy-4-methylsulfoximino-phenyl)-5-carbamido-benzimidazol**

Hergestellt analog Beispiel 7 aus 2-(2-Methoxy-4-methylsulfinyl-phenyl)-5-carbamido-benzimidazolund O-Mesitylen-acethydroxamsäure-äthylester.
Schmelzpunkt: 260-262°C,
Ausbeute: 41,4 % der Theorie.

Analog werden folgende Verbindungen erhalten:

2-(2-Methoxy-4-ethylsulfoximino-phenyl)-5-carbamido-benzimidazol

2-(2-Methoxy-4-n-propylsulfoximino-phenyl)-5-carbamido-benzimidazol

2-(2-Ethoxy-4-methylsulfoximino-phenyl)-5-carbamido-benzimidazol

## Beispiel 11

**2-(2-Methoxy-4-benzyloxy-phenyl)-5-fluor-benzimidazol**

Hergestellt analog Beispiel 1 aus 2-Amino-4-fluor-anilin und 2-Methoxy-4-benzyloxy-benzoesäure in siedendem Phosphoroxychlorid.
Schmelzpunkt: 190-191°C,
Ausbeute: 59,2 % der Theorie.

Analog werden folgende Verbindungen erhalten:

2-(2-Methoxy-4-benzyloxy-phenyl)-5-chlor-benzimidazol

2-(2-Methoxy-4-benzyloxy-phenyl)-5-brom-benzimidazol

## Beispiel 12

### 2-(2-Methoxy-4-hydroxy-phenyl)-5-fluor-benzimidazol

2,1 g 2-(2-Methoxy-4-benzyloxy-phenyl)-5-fluor-benzimidazol werden in 100 ml Ethanol mit 0,2 g Palladium/Kohle (10%ig) bei 20°C und einem Wasserstoffdruck von 4 bar katalytisch hydriert. Danach wird der Katalysator abfiltriert, die klare Lösung eingedampft und der Rückstand durch Verreiben mit Äther zur Kristallisation gebracht.
Schmelzpunkt: 291-293°C,
Ausbeute: 87,7 % der Theorie.

## Beispiel 13

### 2-(2-Methoxy-4-cyanomethoxy-phenyl)-5-fluor-benzimidazol

1,22 g 2-(2-Methoxy-4-hydroxy-phenyl)-5-fluor-benzimidazol werden in 10 ml Dimethylsulfoxid gelöst, mit 0,65 g wasserfreiem Kaliumkarbonat versetzt und dann 0,37 g Chloracetonitril, gelöst in 3 ml Dimethylsulfoxid, bei 20°C innerhalb 35 Minuten unter Rühren zugetropft. Man läßt 14 Stunden rühren, trägt das Reaktionsgemisch in 30 ml Eiswasser ein und stellt mit 2n Essigsäure auf $p_H$ = 5. Das ausgefallene Produkt wird durch Erwärmen auf 70°C kristallin. Man saugt ab, wäscht mit Wasser nach und trocknet die Substanz bei 70°C. Zur Reinigung chromatographiert man das Rohprodukt über eine Kieselgelsäule mit einem Gemisch von Methylenchlorid/Essigester = 5:1.
Schmelzpunkt: 185-190°C,
Ausbeute: 0,68 g (48,7 % der Theorie).

Analog werden folgende Verbindungen erhalten:

2-(2-Methoxy-4-cyanomethylthio-phenyl)-5-fluor-benzimidazol

2-(2-Methoxy-4-allyloxy-phenyl)-5-fluor-benzimidazol

2-(2-Ethoxy-4-cyanomethoxy-phenyl)-5-fluor-benzimidazol

2-(2-Ethoxy-4-allylthio-phenyl)-5-chlor-benzimidazol

2-(2-Methoxy-4-cyanomethoxy-phenyl)-5-brom-benzimidazol

2-(2-Methoxy-4-buten-2-yloxy-phenyl)-5-fluor-benzimidazol

Beispiel 14

2-(2-Methoxy-4-propargyloxy-phenyl)-5-nitro-benzimidazol

Hergestellt analog Beispiel 1 aus 2-Amino-4-nitro-anilin und 2-Methoxy-4-propargyloxy-benzoesäure in siedendem Phosphoroxychlorid.
Schmelzpunkt: 243-245°C,
Ausbeute: 83,1 % der Theorie.

Analog werden folgende Verbindungen erhalten:

2-(2-Methoxy-4-butin-2-yloxy-phenyl)-5-nitro-benzimidazol

2-(2-Ethoxy-4-propargyloxy-phenyl)-5-nitro-benzimidazol

Beispiel 15

2-(2-Methoxy-4-propargyloxy-phenyl)-5-amino-benzimidazol

1,8 g 2-(2-Methoxy-4-propargyloxy-phenyl)-5-nitro-benzimidazol werden in 50 ml Eisessig suspendiert, auf 90°C erhitzt und innerhalb 15 Minuten mit einer Lösung von 9,4 g Natriumdithionit in 50 ml Wasser versetzt. Die entstandene hellbraune Lösung wird am Rotationsverdampfer im Vakuum eingedampft. Der Rückstand wird mit 50 ml Wasser verrührt, bis zur alkalischen Reaktion mit Natriumcarbonat versetzt und 3 mal mit einem Chloroform-Methanol-Gemisch = 4:1 extrahiert. Der gesamte Extrakt wird über Magnesiumsulfat getrocknet, eingedampft und der schaumige Rückstand im Exsikkator getrocknet.
Ausbeute: 0,40 g (4,4 % der Theorie),
$R_f$-Wert: 0,25 (Kieselgel, Fließmittel: Äthylenchlorid/-Äthanol = 9:1).

Analog werden folgende Verbindungen erhalten:

2-(2-Methoxy-4-butin-2-yloxy-phenyl)-5-amino-benzimidazol

2-(2-Ethoxy-4-propargyloxy-phenyl)-5-amino-benzimidazol

## Beispiel 16

2-(2-Methoxy-4-propargyloxy-phenyl)-5-methylaminocarbonyl-amino-benzimidazol

0,36 g 2-(2-Methoxy-4-propargyloxy-phenyl)-5-amino-benzimidazol werden in 5 ml Tetrahydrofuran gelöst, mit 0,14 g Methylisocyanat versetzt und 2 Stunden bei 60°C erhitzt. Danach wird im Vakuum eingedampft. Der Rückstand wird durch Säulenchromatographie über Kieselgel mit Äthylenchlorid/Äthanol = 10:1 gereinigt.
Schmelzpunkt: 203-204°C,
Ausbeute: 0,34 g (78,9 % der Theorie).

Analog werden folgende Verbindungen erhalten:

2-(2-Methoxy-4-butin-2-yloxy-phenyl)-5-methylaminocarbonyl-amino-benzimidazol

2-(2-Ethoxy-4-propargyloxy-phenyl)-5-methylaminocarbonyl-amino-benzimidazol

2-(2-Ethoxy-4-propargyloxy-phenyl)-5-ethylaminocarbonylamino-benzimidazol

## Beispiel 17

**2-(2-Methoxy-4-propargyloxy-phenyl)-5-methoxycarbonyl-benz-imidazol**

Hergestellt analog Beispiel 1 aus 3,4-Diamino-benzoesäuremethylester-dihydrochlorid und 2-Methoxy-4-propargyloxy-benzoesäure siedendem Phosphoroxychlorid.
Schmelzpunkt: 116-120°C,
Ausbeute: 59,5 % der Theorie.

Analog wird folgende Verbindung erhalten:

2-(2-Ethoxy-4-propargyloxy-phenyl)-5-methoxycarbonyl-benzimidazol

## Beispiel 18

**2-(2-Methoxy-4-propargyloxy-phenyl)-5-carboxy-benzimidazol**

Hergestellt analog Beispiel 2 aus 2-(2-Methoxy-4-propargyloxy-phenyl)-5-methoxycarbonyl-benzimidazol und 2n Natronlauge.
Schmelzpunkt: 235-240°C,
Ausbeute: 92,8 % der Theorie.

Analog werden folgende Verbindungen erhalten:

2-(2-Ethoxy-4-propargyloxy-phenyl)-5-carboxy-benzimidazol

2-(2-Methoxy-4-allyloxy-phenyl)-5-carboxy-benzimidazol

## Beispiel 19

**2-(2-Methoxy-4-propargyloxy-phenyl)-5-aminocarbonyl-benzimidazol**

Hergestellt nach Beispiel 3 aus 2-(2-Methoxy-4-propargyloxy-phenyl)-5-carboxy-benzimidazol und Thionylchlorid und anschließende Umsetzung des entstandenen 2-(2-Methoxy-4-propargyloxy-phenyl)-5-chlorcarbonyl-benzimidazols mit konzentriertem Ammoniak. Das Rohprodukt wird mittels Säulenchromatographie über Kieselgel mit Äthylenchlorid/Äthanol = 9:1 gereinigt.
Schmelzpunkt: 150-152°C,
Ausbeute: 35,0 % der Theorie.

Analog werden folgende Verbindungen erhalten:

2-(2-Methoxy-4-propargyloxy-phenyl)-5-methylaminocarbonyl-benzimidazol

2-(2-Ethoxy-4-propargyloxy-phenyl)-5-aminocarbonyl-benzimidazol

2-(2-Methoxy-4-allyloxy-phenyl)-5-aminocarbonyl-benzimidazol

2-(2-Methoxy-4-allyloxy-phenyl)-5-n-propylamino-carbonyl-benzimidazol

Beispiel 20

2-(2-Methoxy-4-propargyloxy-phenyl)-5-cyano-benzimidazol

Hergestellt analog Beispiel 1 aus 2-Amino-4-cyano-anilin und 2-Methoxy-4-propargyloxy-benzoesäure in siedendem Phosphoroxychlorid.
Schmelzpunkt: 243-245°C,
Ausbeute: 85,0 % der Theorie.

Analog wird folgende Verbindung erhalten:

2-(2-Ethoxy-4-propargyloxy-phenyl)-5-cyano-benzimidazol

Beispiel 21

2-(2-Methoxy-4-aminosulfonyl-phenyl)-5-cyano-benzimidazol

a) 1,47 g 2-Nitro-4-cyano-anilin und 1,96 g 2-Methoxy-4-aminosulfonyl-benzoylchlorid werden in 50 ml trockenem Chlorbenzol 5 Stunden lang am Rückfluß zum Sieden erhitzt. Aus der ursprünglich klaren Lösung scheiden sich während dieser Zeit Kristalle von (2-Methoxy-4-aminosulfonyl)-benz-(2-nitro-4-cyano)-anilid ab. Man saugt noch heißt ab, wäscht mit wenig kaltem Chlorbenzol und dann mit Essigester nach und trocknet bei 60°C.
Schmelzpunkt: 270-274°C,
Ausbeute: 2,47 g (72,9 % der Theorie).

b) 2,40 g der gemäß Beispiel a) erhaltenen Substanz werden in 250 ml Eisessig aufgeschlämmt, mit 20 g Eisenpulver versetzt und 1,5 Stunden am Rückfluß zum Sieden erhitzt. Man filtriert von dem Eisenrückstand ab, destilliert den Eisessig im Vakuum ab und verrührt den Rückstand mit Wasser. Die ausgeschiedenen Kristalle werden abgesaugt und an einer

Kieselgelsäule mit Methylenchlorid/Essigester = 1:1 chromatographiert. Man erhält weiße Kristalle von 2-(2-Methoxy-4-aminosulfonyl-phenyl)-5-cyano-benzimidazol.
Schmelzpunkt: 284-286°C,
Ausbeute: 0,455 g (21,7 % der Theorie).

Analog werden folgende Verbindungen erhalten:

2-(2-Methoxy-4-methylaminosulfonyl-phenyl)-5-cyano-benzimidazol

2-(2-Methoxy-4-dimethylaminosulfonyl-phenyl)-5-cyano-benzimidazol

2-(2-Ethoxy-4-aminosulfonyl-phenyl)-5-cyano-benzimidazol

2-(2-Ethoxy-4-methylaminosulfonyl-phenyl)-5-cyano-benzimidazol

2-(2-Ethoxy-4-dimethylaminosulfonyl-phenyl)-5-cyano-benzimidazol

2-(2-Methoxy-4-n-propylaminosulfonyl-phenyl)-5-cyano-benzimidazol

2-(2-Methoxy-4-di-n-propylaminosulfonyl-phenyl)-5-cyano-benzimidazol

## Beispiel 22

**2-(2-Methoxy-4-aminosulfonyl-phenyl)-5-methoxycarbonyl-benzimidazol**

Hergestellt analog Beispiel 1 aus 2-Methoxy-4-aminosulfonyl-benzoesäure und 3,4-Diamino-benzoesäuremethylester in siedendem Phosphoroxychlorid.
Schmelzpunkt: 284-286°C,
Ausbeute: 48,4 % der Theorie.

Analog werden folgende Verbindungen erhalten:

2-(2-Methoxy-4-methylaminosulfonyl-phenyl)-5-methoxycarbonyl-benzimidazol

2-(2-Methoxy-4-dimethylaminosulfonyl-phenyl)-5-methoxycarbonyl-benzimidazol

2-(2-Ethoxy-4-di-n-propylaminosulfonyl-phenyl)-5-methoxycarbonyl-benzimidazol

## Beispiel 23

### 2-(2-Methoxy-4-aminosulfonyl-phenyl)-5-carboxy-benzimidazol

Hergestellt analog Beispiel 2 aus 2-(2-Methoxy-4-aminosulfonyl-phenyl)-5-methoxycarbonyl-benzimidazol mit 2n Natronlauge.
Schmelzpunkt: 321-323°C,
Ausbeute: 61,5 % der Theorie.

Analog werden folgende Verbindungen erhalten:

2-(2-Methoxy-4-methylaminosulfonyl-phenyl)-5-carboxy-benzimidazol

2-(2-Methoxy-4-dimethylaminosulfonyl-phenyl)-5-carboxy-benzimidazol

## Beispiel 24

**2-(2-Methoxy-4-aminosulfonyl-phenyl)-5-aminocarbonyl-benzimidazol**

Hergestellt analog Beispiel 3 aus 2-(2-Methoxy-4-aminosulfonyl-phenyl)-5-carboxy-benzimidazol durch Reaktion mit Thionylchlorid und dann mit konzentriertem Ammoniak.
Schmelzpunkt: 311-313°C,
Ausbeute: 76,0 % der Theorie.

Analog werden folgende Verbindungen erhalten:

2-(2-Methoxy-4-aminosulfonyl-phenyl)-5-methylaminocarbonyl-benzimidazol

2-(2-Methoxy-4-methylaminosulfonyl-phenyl)-5-amino-carbonyl-benzimidazol

2-(2-Methoxy-4-dimethylaminosulfonyl-phenyl)-5-aminocarbonyl-benzimidazol

2-(2-Methoxy-4-di-n-propylaminosulfonyl-phenyl)-5-aminocarbonyl-benzimidazol

2-(2-Methoxy-4-aminosulfonyl-phenyl)-5-di-n-propylaminocarbonyl-benzimidazol

## Beispiel 25

**2-(2-Methoxy-4-aminosulfonyl-phenyl)-5-aminocarbonyl-benzimidazol**

0,657 g 2-(2-Methoxy-4-aminosulfonyl-phenyl)-5-cyano-benzimidazol werden in 2 ml konzentrierter Schwefelsäure portionsweise eingetragen und bei 30°C 24 Stunden stehen gelassen. Dann zerlegt man mit Eis, saugt die ausgeschiedenen Kristalle ab und wäscht säurefrei.

Schmelzpunkt: 311,5-313,5°C,

Ausbeute: 60,0 % der Theorie.

## Beispiel A

**Tabletten zu 100 mg 2-(2-Methoxy-4-propargyloxy-phenyl)-5-cyano-benzimidazol**

Zusammensetzung
1 Tablette enthält:

| | |
|---|---|
| Wirksubstanz | 100,0 mg |
| Milchzucker | 50,0 mg |
| Polyvinylpyrrolidon | 5,0 mg |
| Carboxymethylcellulose | 19,0 mg |
| Magnesiumstearat | 1,0 mg |
| | 175,0 mg |

Feuchtsiebung: 1,5 mm
Trocknen: Umlufttrockenschrank 50°C
Trockensiebung: 1 mm
Dem Granulat die restlichen Hilfsstoffe zumischen und Endmischung zu Tabletten verpressen.
Tablettengewicht: 175 mg
Stempel: 8 mm

## Beispiel B

**Dragées zu 50 mg 2-(2-Methoxy-4-propargyloxy-phenyl)-5-cyano-benzimidazol**

Zusammensetzung:
1 Dragéekern enthält:

| | |
|---|---|
| Wirksubstanz | 50,0 mg |
| Maisstärke getr. | 20,0 mg |
| Lösliche Stärke | 2,0 mg |
| Carboxymethylcellulose | 7,0 mg |
| Magnesiumstearat | 1,0 mg |
| | 80,0 mg |

Wirkstoff und Stärke mit wäßriger Lösung der löslichen Stärke gleichmäßig befeuchten.

| | |
|---|---|
| Feuchtsiebung: | 1,0 mm |
| Trockensiebung: | 1,0 mm, |
| Trocknung: | 50°C im Umlufttrockenschrank |

Granulat und restliche Hilfsstoffe mischen und zu Kernen verpressen.

| | |
|---|---|
| Kerngewicht: | 80 mg |
| Stempel: | 6 mm |
| Wölbungsradius: | 5 mm |

Die fertigen Kerne werden auf übliche Weise mit einem Zuckerüberzug im Dragierkessel versehen.

Dragéegewicht: 120 mg

## Beispiel C

**Suppositorien zu 75 mg 8-(2-Methoxy-4-propargyloxyphenyl)-5-cyano-benzimidazol**

1 Zäpfchen enthält:

| | |
|---|---|
| Wirksubstanz | 75,0 mg |
| Zäpfchenmasse (z.B. Witepsol H 19 und Witepsol W 45) | 1 625,0 mg |
| | 1 700,0 mg |

## Herstellungsverfahren:

Die Zäpfchenmasse wird geschmolzen. Bei 38°C wird die gemahlene Wirksubstanz in der Schmelze homogen dispergiert. Es wird auf 35°C abgekühlt und in vorgekühlte Suppositorienformen ausgegossen.

Zäpfchengewicht: 1,7 g

Beispiel D

Ampullen zu 50 mg 2-(2-Methoxy-4-propargyloxy-phenyl)-5-cyano-benzimidazol

1 Ampulle enthält:

| | |
|---|---|
| Wirksubstanz | 50,0 mg |
| Ethoxylierte Hydroxystearinsäure | 750,0 mg |
| 1,2-Propylenglykol | 1000,0 mg |
| Dest. Wasser ad | 5,0 ml |

Herstellungsverfahren:

Die Wirksubstanz wird in 1,2-Propylenglykol und ethoxylierter Hydroxystearinsäure gelöst, dann mit Wasser auf das angegebene Volumen aufgefüllt und steril filtriert.
Abfüllung: in Ampullen zu 5 ml
Sterilisation: 20 Minuten bei 120°C

Beispiel E

Tropfen zu 100 mg 2-(2-Methoxy-4-propargyloxy-phenyl)-5-cyano-benzimidazol

| | | | |
|---|---|---|---|
| Wirksubstanz | | 1,0 | g |
| p-Oxybenzoesäuremethylester | | 0,035 | g |
| p-Oxybenzoesäurepropylester | | 0,015 | g |
| Anisöl | | 0,05 | g |
| Menthol | | 0,06 | g |
| Saccharin-Natrium | | 1,0 | g |
| Glycerin | | 10,0 | g |
| Ethanol | | 40,0 | g |
| Dest. Wasser | ad | 100,0 | ml |

Herstellungsverfahren:

Die Benzoesäureester werden in Ethanol gelöst und anschließend das Anisöl und das Menthol zugegeben. Dann wird die Wirksubstanz, Glycerin und Saccharin-Natrium im Wasser gelöst zugegeben. Die Lösung wird anschließend klar filtriert.

## Patentansprüche

1. Benzimidazole der allgemeinen Formel

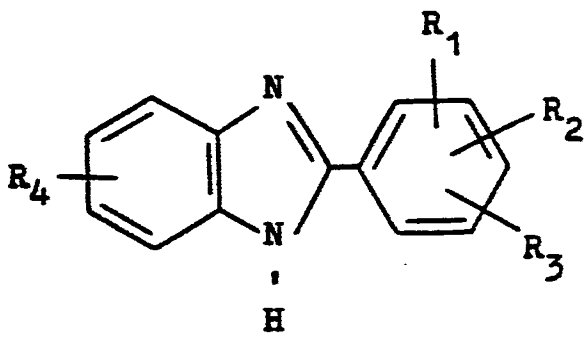

,(I)

in der

$R_1$ eine Hydroxy-, Alkoxy-, Phenylalkoxy-, Cyanalkoxy-, Alkylsulfenyl-, Alkylsulfinyl-, Alkylsulfonyl-, Aminosulfonyl-, Alkylaminosulfonyl-, Dialkylaminosulfonyl-, Alkylsulfoximino-, Alkenyloxy- oder Alkinyloxygruppe,

$R_2$ ein Wasserstoffatom, eine Hydroxy-, Alkoxy-, Phenylalkoxy-, Amino-, Alkylamino- oder Dialkylaminogruppe,

$R_3$ ein Wasserstoffatom oder eine Alkoxygruppe und

$R_4$ ein Wasserstoff- oder Halogenatom, eine Cyan-, Nitro-, Amino-, Carboxy-, Alkoxycarbonyl-, Aminocarbonyl-, Alkylaminocarbonyl-, Dialkylaminocarbonyl-, Aminocarbonylamino-, Alkylaminocarbonylamino- oder Dialkylaminocarbonylaminogruppe, wobei der Alkylteil jeweils 1 bis 3 Kohlenstoffatome und der Alkenyl- oder Alkinylteil jeweils 3 bis 5 Kohlenstoffatome enthalten kann, bedeuten, deren Tautomere und deren Säureadditionssalze.

2. Benzimidazole der allgemeinen Formel I gemäß Anspruch 1, in der $R_1$ und $R_4$ wie im Anspruch 1 definiert sind, $R_2$ mit Ausnahme des Wasserstoffatoms die für $R_2$ eingangs erwähnten Bedeutungen besitzt und $R_3$ ein Wasserstoffatom darstellt, deren Tautomere und deren Säureadditionssalze.

3. Benzimidazole der allgemeinen Formel I gemäß Anspruch 1, in der $R_1$ bis $R_3$ wie im Anspruch 2 definiert sind, $R_1$ in 4-Stellung und $R_2$ in 2-Stellung steht und $R_4$ mit Ausnahme des Wasserstoffatoms die für $R_4$ im Anspruch 1 eingangs erwähnten Bedeutungen besitzt, deren Tautomere und deren Säureadditionsalze.

4. Benzimidazole der allgemeinen Formel

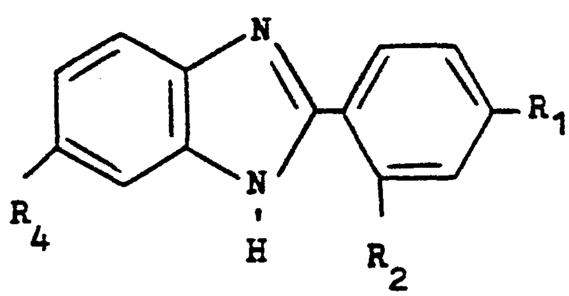

,(Ia)

in der $R_1$ eine Hydroxy-, Benzyloxy-, Allyloxy-, Propargyloxy-, Alkylsulfenyl-, Alkylsulfinyl-, Alkylsulfonyl-, Alkylsulfoximino- oder Aminosulfonylgruppe,

$R_2$ eine Alkoxygruppe und

$R_4$ ein Fluor-, Chlor- oder Bromatom, eine Cyan-, Carboxy-, Aminocarbonyl-, Alkoxycarbonyl-, Nitro- oder Aminogruppe bedeuten, wobei der Alkylteil jeweils 1 oder 2 Kohlenstoffatome enthalten kann, deren Tautomere und deren Säureadditionssalze.

5. 2-(2-Methoxy-4-methylsulfonyl-phenyl)-5-cyano-benzimidazol, dessen Tautomere und dessen Säureadditionssalze.

6. 2-(2-Methoxy-4-propargyloxy-phenyl)-5-cyano-benzimidazol, dessen Tautomere und dessen Säureadditionssalze.

7. Physiologisch verträgliche Säureadditionssalze der Verbindungen gemäß den Ansprüchen 1 bis 6.

8. Arzneimittel, enthaltend eine Verbindung gemäß den Ansprüchen 1 bis 6 oder ein physiologisch verträgliches Säureadditionssalz gemäß Anspruch 7 neben einem oder mehreren inerten Trägerstoffen und/oder Verdünnungsmitteln.

9. Verwendung einer Verbindung gemäß den Ansprüchen 1-6 oder eines physiologisch verträglichen Säureadditionssalzes gemäß Anspruch 7 zur Behandlung von Herzinsuffizienzen.

10. Verfahren zur Herstellung eines Arzneimittels gemäß Anspruch 8, dadurch gekennzeichnet, daß auf nichtchemischem Wege eine Verbindung gemäß den Ansprüchen 1-6 oder ein physiologisch verträgliches Säureadditionssalz gemäß Anspruch 7 in einen oder mehrere inerte Trägerstoffe und/oder Verdünnungsmittel eingearbeitet wird.

11. Verfahren zur Herstellung der Verbindungen gemäß den Ansprüchen 1-7 dadurch gekennzeichnet, daß

a) eine gegebenenfalls im Reaktionsgemisch hergestellte Verbindung der allgemeinen Formel

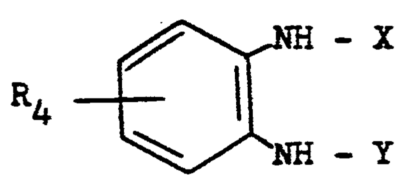

,(II)

in der $R_4$ wie im Anspruch 1 definiert ist, einer der Reste X oder Y ein Wasserstoffatom und der andere der beiden Reste X und Y oder beide Reste X und Y eine Gruppe der Formel

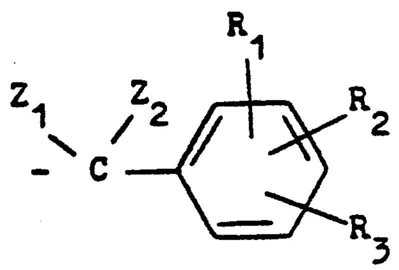

darstellen, in der

$R_1$ bis $R_3$ wie im Anspruch 1 definiert sind, $Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder

$Z_1$ und $Z_2$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, cyclisiert wird oder

b) zur Herstellung von Verbindungen der allgemeinen Formel I, in der $R_1$ eine Alkylsulfinyl- oder Alkylsulfonylgruppe darstellt, eine Verbindung der allgemeinen Formel

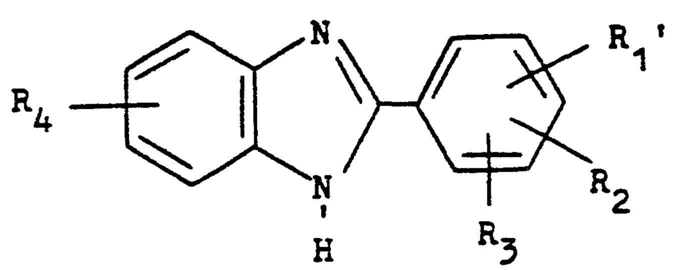

,(III)

in der
$R_2$ bis $R_4$ wie im Anspruch 1 definiert sind und
$R_1$' eine Alkylsulfenyl- oder Alkylsulfinylgruppe mit jeweils 1 bis 3 Kohlenstoffatomen im Alkylteil darstellt, oxidiert wird oder

c) zur Herstellung von Verbindungen der allgemeinen Formel I, in der $R_1$ eine Alkoxy-, Phenylalkoxy-, Cyanalkoxy-, Alkylsulfenyl-, Alkenyloxy- oder Alkinyloxygruppe darstellt, eine Verbindung der allgemeinen Formel

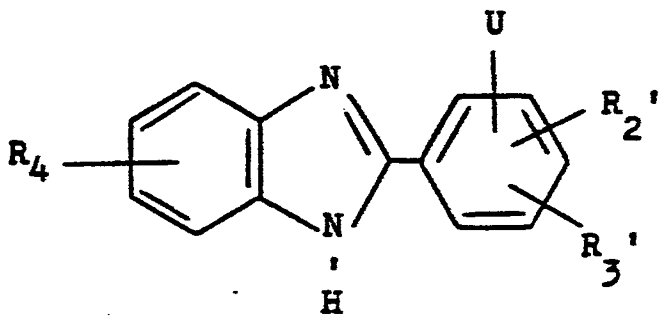

,(IV)

in der
$R_4$ wie im Anspruch 1 definiert ist,
U eine Hydroxy- oder Mercaptogruppe darstellt,
$R_2$' eine Hydroxygruppe oder die für $R_2$ im Anspruch 1 erwähnten Bedeutungen besitzt und
$R_3$' eine Hydroxygruppe oder die für $R_3$ im Anspruch 1 erwähnten Bedeutungen besitzt, mit einem Halogenid der allgemeinen Formel

$$W - R_5 \qquad ,(V)$$

in der
$R_5$ eine Alkyl-, Phenylalkyl-, Cyanalkyl-, Alkenyl- oder Alkinylgruppe, wobei der Alkylteil jeweils 1 bis 3 Kohlenstoffatomen und der Alkenyl- bzw. Alkinylteil jeweils 3 bis 5 Kohlenstoffatomen enthalten kann, und
W eine nukleophile Austrittsgruppe wie ein Chlor-, Brom- oder Jodatom darstellen, umgesetzt wird oder

d) zur Herstellung von Verbindungen der allgemeinen Formel I, in der $R_4$ eine Alkoxcarbonyl-, Aminocarbonyl-, Alkylaminocarbonyl- oder Dialkylaminocarbonylgruppe darstellt, ein Benzimidazol der allgemeinen Formel

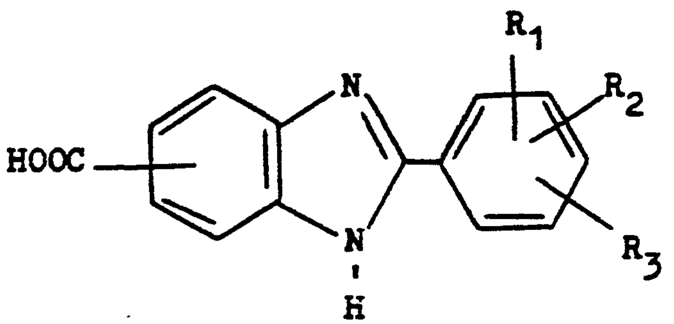

,(VI)

in der
$R_1$ bis $R_3$ wie im Anspruch 1 definiert sind, oder deren gegebenenfalls im Reaktionsgemisch hergestelltes reaktionsfähiges Derivat mit einer Verbindung der allgemeinen Formel

$$H - R_6 \quad ,(VII)$$

in der
$R_6$ eine Alkoxy-, Amino-, Alkylamino- oder Dialkylaminogruppe darstellt, wobei der Alkylteil jeweils 1 bis 3 Kohlenstoffatomen enthalten kann, oder mit einem gegebenenfalls im Reaktionsgemisch gebildeten N-aktivierten Amin der allgemeinen Formel VII, wenn eine Carbonsäure der allgemeinen Formel VI eingesetzt wird, umgesetzt wird oder

e) zur Herstellung von Verbindungen der allgemeinen Formel I, in der $R_1$ eine Alkylsulfoximinogruppe darstellt, ein Sulfoxid der allgemeinen Formel

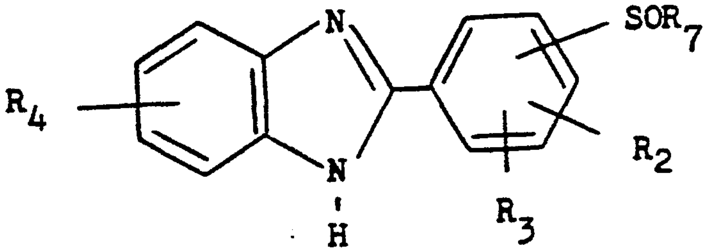

,(VIII)

0148431

in der
$R_2$ bis $R_4$ wie im Anspruch 1 definiert sind und
$R_7$ eine Alkylgruppe mit 1 bis 3 Kohlenstoffatomen darstellt, mit gegebenenfalls im Reaktionsgemisch gebildeter Stickstoffwasserstoffsäure umgesetzt wird oder

f) zur Herstellung von Verbindungen der allgemeinen Formel I, in der $R_1$ eine Alkylsulfoximinogruppe darstellt, ein Sulfoxid der allgemeinen Formel

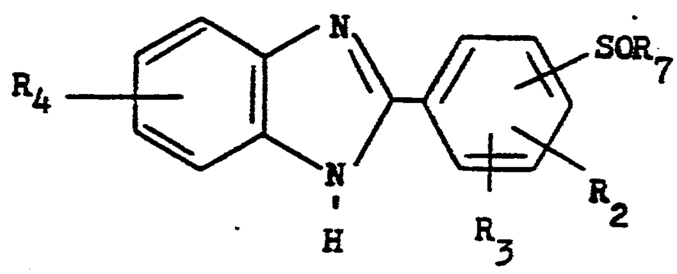

,(VIII)

in der
$R_2$ bis $R_4$ wie im Anspruch 1 definiert sind und
$R_7$ eine Alkylgruppe mit 1 bis 3 Kohlenstoffatomen darstellt, mit einer Verbindung der allgemeinen Formel

$$H_2N - O - W - R_8 \quad ,(IX)$$

in der
W eine Carbonyl- oder Sulfonylgruppe und
$R_8$ eine in o-Stellung disubstituierte Arylgruppe wie eine 2,4,6-Trimethylphenyl- oder 2,4,6-Triisopropylphenylgruppe darstellen, umgesetzt wird

und gewünschtenfalls anschließend eine so erhaltene Verbindung der allgemeinen Formel I, in der $R_4$ eine Alkoxycarbonyl-, Cyan-, Aminocarbonyl-, Alkylaminocarbonyl- oder Dialkylaminocarbonylgruppe darstellt, mittels Hydroylse in eine entsprechende Verbindung der allgemeinen Formel I, in der $R_4$ die Carboxylgruppe darstellt, übergeführt wird und/oder

eine erhaltene Verbindung der allgemeinen Formel I, in der $R_4$ eine Aminocarbonylgruppe darstellt, mittels Dehydratisierung in eine entsprechende Verbindung der allgemeinen Formel I, in der $R_4$ die Cyangruppe darstellt, übergeführt wird und/oder

eine erhaltene Verbindung der allgemeinen Formel I, in der $R_4$ eine Nitrogruppe darstellt, mittels Reduktion in eine entsprechende Verbindung der allgemeinen Formel I, in der $R_4$ eine Aminogruppe darstellt, übergeführt wird und/oder

eine erhaltene Verbindung der allgemeinen Formel I, in der $R_4$ eine Aminogruppe darstellt, mittels Carbamoylierung in eine entsprechende Verbindung der allgemeinen Formel I, in der $R_4$ eine Aminocarbonylamino-, Alkylaminocarbonylamino- oder Dialkylaminocarbonylaminogruppe darstellt, übergeführt wird und/oder

eine erhaltene Verbindung der allgemeinen Formel I, in der $R_1$ und/oder $R_2$ eine Benzyloxygruppe darstellen, mittels Entbenzylierung in eine entsprechende Verbindung der allgemeinen Formel I, in der $R_1$ und/oder $R_2$ eine Hydroxygruppe darstellen, übergeführt wird und/oder

eine erhaltene Verbindung der allgemeinen Formel I in ihr Säureadditionssalz, insbesondere in ihr physiologisch verträgliches Säureadditionssalz mit einer anorganischen oder organischen Säure, übergeführt wird.

0148431

Nummer der Anmeldung

Europäisches Patentamt

# EUROPÄISCHER RECHERCHENBERICHT

EP 84 11 4999

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,X | EP-A-0 098 448 (DR. K. THOMAE GMBH)<br>* Ansprüche 1, 12-15; Beispiele A, B * | 1-4,7-11 | C 07 D 235/18<br>A 61 K 31/415 |
| | --- | | |
| X | EP-A-0 001 246 (CASSELLA AG.)<br>* Ansprüche 3, 4; Seite 21, Tabelle, 1., 2. Formel * | 1,2,11 | |
| | --- | | |
| X | FR-M- 5 471 (MERCK AND CO., INC:)<br>* Ansprüche; Seite 5, Tabelle, 23., 26., 37., 39. Verbindungen * | 1,7,8 | |
| | --- | | |
| X | FR-A-1 569 337 (MANUFACTURES J.R. BOTTU)<br>* Seite 1, letzter Absatz * | 1 | **RECHERCHIERTE SACHGEBIETE (Int. Cl.4)** |
| | --- | | |
| X | FR-A-1 241 329 (CIBA S.A.)<br>* Seite 4, Formel (5) * | 1 | C 07 D 235/00 |
| | --- | | |
| X | DE-B-1 447 733 (HOECHST AG.)<br>* Spalten 9, 10, Formel Nr. 2 * | 1 | |
| | --- -/- | | |

Der vorliegende Recherchenbericht wurde für alle Patentansprüche erstellt.

| Recherchenort | Abschlußdatum der Recherche | Prüfer |
|---|---|---|
| BERLIN | 13-03-1985 | HASS C V F |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03.82

0148431
Nummer der Anmeldung

Europäisches Patentamt

# EUROPÄISCHER RECHERCHENBERICHT

EP 84 11 4999

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 56, Nr. 7, 2. April 1962, Columbus, Ohio, USA; T. BACCHETTI et al. "Decarboxylation of 1,2,4-oxadiazol-5-ones. Syntheses of benzimidazoles. II", Spalte 7304 c-g & Atti Accad. Nazl. Lincei, Rend., Classe Sci. Fis Seiten 824-835 | 1 | |
| | --- | | |
| D,A | EP-A-0 022 495 (DR. K. THOMAE GMBH) * Ansprüche 1-3, 8-10; Beispiele A-E * | 1,7-11 | |
| | ----- | | |

RECHERCHIERTE SACHGEBIETE (Int. Cl.4)

Der vorliegende Recherchenbericht wurde für alle Patentansprüche erstellt.

| Recherchenort | Abschlußdatum der Recherche | Prüfer |
|---|---|---|
| BERLIN | 13-03-1985 | HASS C V F |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82